# EUROPEAN PATENT APPLICATION

(11) **EP 1 892 042 A1**
(43) Date of publication of application: **27.02.2008**
(21) Application number: 06119439.5
(22) Date of filing: 24.08.2006
(51) Int. Cl.: B01J 31/06, B01J 31/02, C07D 207/16

(54) **Proline derivatives supported on polymers through triazole or tetrazole linkers for organocatalytic applications**

(71) Applicant: Institut Catala D'Investigacio Quimica, 43007 Tarragona (ES)
(72) Inventor: PERICÀS BRONDO, Miquel A., 43007 Tarragona (ES); JIMENO MOLLET, Ciril, 43007 Tarragona (ES); FONT SEGURA, Daniel, 43007 Tarragona (ES)
(74) Representative: Barlocci, Anna

(57) **Abstract**

The present invention relates to novel organocatalysts for carrying out asymmetric reactions, which are proline derivatives supported on polymers. through triazole or tetrazole linkers. The organocatalysts show a high purity and a high stereoselectivity, and excellent results has been obtained not only with organic solvents but also with water as solvent.

## Description

The present invention is directed to organocatalysts. Particularly to proline derivatives supported on polymers through triazole or tetrazole linkers for organocatalytic applications, processes for their preparation, and uses thereof.

### BACKGROUND ART

Asymmetric organocatalysis has become a field of central importance for the synthesis of chiral molecules. The primary advantage of this technique is that it avoids the use of metals, which can be both expensive and toxic.

Compared with their counterpart organometallic catalysts, organocatalysts afford distinguishable benefits. First, they are easily prepared and handled, more environmentally benign and cheaper since they do not rely on expensive and toxic metals. Second, generally organocatalyst catalyzed reactions can be performed under an aerobic condition in common, even water containing, organic solvents. Third, they are more robust and can be stored and handled in an air atmosphere, thus providing operational simplicity. Fourth, these small organic molecules can be immobilized on a solid support and reused more conveniently than are organometallic / bioorganic analogues. Fifth, it has been demonstrated that, in many cases, these small molecule catalysts display high catalytic activity for organic reactions that proceed with excellent enantio- and/or diastereoselectivity.

Other advantages associated with the use of organocatalysts, especially compared with enzymes and other bioorganic catalysts, are that they are more stable and less expensive and that they are capable of catalyzing a variety of organic reactions with a diverse range of different substrates.

Proline is one example of a versatile organocatalyst capable of effecting a variety of catalytic asymmetric transformations, which despite having been used effectively in the Hajos-Parrish-Eder-Sauer-Wiechert reaction in the 1970's, has only recently received full attention in synthetic applications, such as aldol, Mannich, Michael, Robinson annulation, nitro-Michael, direct electrophilic α-amination reactions, as well as other asymmetric transformations such as allylic oxidations, transfer-hydrogenations, and Diels-Alder type reactions. However, there are a number of drawbacks in the use of proline. First, its limited solvent compatibility; often reactions are performed in very polar solvents such as DMSO, MeOH, or H₂O, and the enantioselectivity obtained is not always the desired. Secondly, a relatively high catalyst loading is usually required to effect the desired reaction in a reasonable timescale; commonly proline is used at levels of around 20 mol%. Furthermore, some reactions such as direct aldol reactions, when catalyzed by proline in water as solvent, affords the racemate.

It has been recently shown that asymmetric direct aldol reactions can be efficiently carried out in water as the sole solvent using as catalysts proline derivatives that contain large apolar groups and behave as aldolase mimics.

Following initial attempts to immobilize proline, excellent results have been achieved using soluble poly(ethylene glycol) (PEG) supported proline (M. Benaglia et al., Adv. Synth. Catal. 2001, vol. 343, pp.171-173; M. Benaglia et al., G. Adv. Synth. Catal. 2002, vol. 344, pp. 533-542), or mesoporous silica supported proline (F. Calderón et al., Adv. Synth. Catal. 2005, vol.347, pp.1395-1403) in organic solvents. However, attempts to work in water with peptides supported on PEG-Polystyrene (PEG-PS) resins have led only to low enantioselectivities (K. Akagawa et al., Tetrahedron Lett. 2005, vol. 46, pp. 8185-8187).

From a practical perspective, the use of water as the solvent in chemical reactions is a very favourable characteristic, but it would be even more desirable to be able to efficiently recycle and reuse the catalysts that perform the reaction in that media.

When a catalyst remains within a reaction mixture it may promote further reactions to breakdown the desired product or to form undesirable side-products within the product mixture. Moreover, the use of monomeric, soluble catalysts renders necessary the introduction of work-up stages aimed to catalyst separation and recovery, thus preventing the operation of continuous processes. It is therefore desirable to provide a heterogeneous supported proline based organocatalyst to overcome the known disadvantages of homogeneous catalysis.

### SUMMARY OF THE INVENTION

Inventors have found a new organocatalyst for asymmetric reactions that is immobilized on a solid support, thereby is efficiently recycled and reused, which shows a high purity and which shows a high stereoselectivity. The new organocatalysts are proline derivatives supported on polymers through triazole or tetrazole linkers. Compared with other immobilized proline derivatives known in the art, excellent results has been obtained not only with organic solvents but also with water as a solvent, thus is specially advantageous for carrying out asymmetric reactions in water as solvent. Thus, the organocatalyst of the invention is a stable compound, which is not degradated by water, maintains its activity during a long time and has a high purity.
According to one aspect, the invention provides an enantiomerically pure supported proline derivative organocatalyst or a salt thereof, or a N-oxide thereof, or a solvate thereof. The enantiomerically pure catalyst according to the invention may be represented by the formula (I) 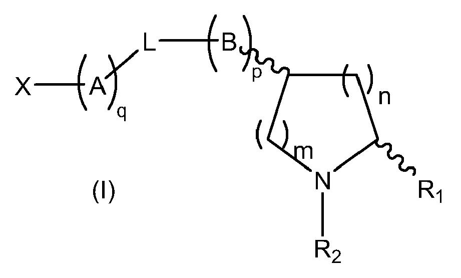 wherein:
the wavy line means any of the two possible configurations of the attached chiral atom;
X represents the polymeric support;
m and n are independently an integer from 0 to 4; preferably when m is 0, n is ≥ 2, and when n is 0, m ≥ 2;
p and q are independently an integer from 0 to 1;
L is a biradical selected from the group consisting of 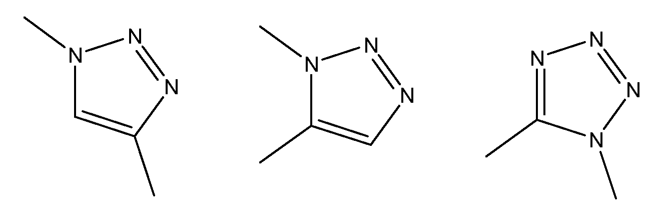 wherein L may be linked to A and B in its two possible positions;
A and B are biradicals independently selected from the group consisting of -alkyl(C₁-C₁₀)-, -O-alkyl(C₁-C₁₀)-, -alkyl(C₁-C₁₀)-O-, and -alkyl(C₁-C₁₀)-O-alkyl(C₁-C₁₀)-;
R₁ is selected from the group consisting of -COR₃, -CONHR₄, cycloalkyl(C₃-C₈), haloalkyl(C₁-C₆), -O-alkyl(C₁-C₆), -alkyl(C₁-C₆)-OH, -alkyl(C₁-C₆)-O-alkyl(C₁-C₆), aryl(C₆-C₁₈), phenylalkyl(C₁-C₆), biphenylalkyl(C₁-C₆), alkyl(C₁-C₆)phenyl, alkyl(C₁-C₆)biphenyl, substituted alkyl(C₁-C₆)biphenyl, phenylalkenyl(C₂-C₆), napthyl, substituted napthyl, napthylalkyl(C₁-C₆), napthylaikenyl(C₂-C₆), morpholyl, morpholylalkyl(C₁-C₆), furyl, substituted furyl, benzofuryl, substituted benzofuryl, pyrrolyl, substituted pyrrolyl, isoxazolyl, substituted isoxazolyl, benzoisoxazolyl, substituted benzoisoxazolyl, imidazolyl, substituted imidazolyl, benzimidazolyl, substituted benzimidazolyl, indolyl, substituted indolyl, pyrazolyl, substituted pyrazolyl, thienyl, substituted thienyl, benzothienyl, substituted benzothienyl, thiazolyl, substituted thiazolyl, benzothiazolyl, substituted benzothiazolyl, quinolinyl, substituted quinolinyl, isoquinolinyl, substituted isoquinolinyl, piperazinyl, piperidinyl, triazolyl, triazolylalkyl(C₁-C₆), tetrazolyl, tetrazolylalkyl(C₁-C₆), diarilhydroxypirrolidonyl, prolinolyl, alkyl(C₁-C₆)prolinol ether, pyridyl and substituted pyridyl;
R₂ is selected from the group consisting of hydrogen and linear or branched alkyl(C₁-C₆);
R₃ is selected from the group consisting of H, OH, linear or branched alkyl(C₁-C₆), alkenyl(C₂-C₆), alkynyl(C₂-C₆), cycloalkyl(C₃-C₈), haloalkyl(C₁-C₆), -O-alkyl(C₁-C₆), aryl(C₆-C₁₈), phenylalkyl(C₂-C₆), furyl, substituted furyl, pyrrolyl, substituted pyrrolyl, isoxazolyl, substituted isoxazolyl, imidazolyl, substituted imidazolyl, indolyl, substituted indolyl, pyrazolyl, substituted pyrazolyl, thienyl, substituted thienyl, thiazolyl, substituted thiazolyl, quinolinyl, substituted quinolinyl, isoquinolinyl, substituted isoquinolinyl, triazolyl, triazolylalkyl(C₁-C₆), tetrazolyl, tetrazolylalkyl(C₁-C₆), -alkyl(C₁-C₆)-O-alkyl(C₁-C₆), morpholyl, morpholylalkyl(C₁-C₆), piperazinyl, piperidinyl, diarilhydroxypirrolidonyl, prolinolyl, alkyl(C₁-C₆)prolinol ether, pyrrolidinyl, pyridyl and substituted pyridyl;
   and
R₄ is selected from the group consisting of hydrogen, linear or branched alkyl(C₁-C₆), cycloalkyl(C₃-C₆), pyridyl, substituted pyridyl, and alkyl(C₁-C₆)Sulfonyl;
the substituents being a radical independently selected from the group consisting of halogen, (C₁-C₄)-alkyl, (C₁-C₄)-alkoxy, and hydroxy;
or a salt thereof, or a N-oxide thereof, or a solvate thereof.

According to another aspect, the invention provides a method for the preparation of the supported proline derivative organocatalysts of the invention, said method comprising:
a) reacting a support having a terminal azide moiety and an proline derivative of formula IIa having a terminal alkyne or nitrile moiety 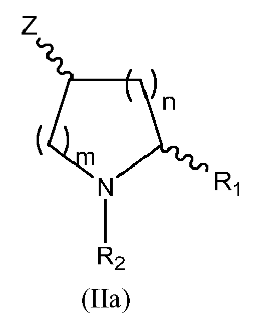 wherein the wavy line means any of the two possible configurations of the attached chiral atom; Z is selected from the group consisting of alkyne(C₃-C₁₀) or alkyl(C₁-C₁₀)-CN; and wherein m, n, R₁ and R₂ are the same as defined above; provided that when R₁ is a reacting group containing an active moiety such as a -COOH, -CONH₂, or -OH, then the active moiety of R₁ must be previously protected with a suitable protecting group, and provided that when R₂ is H, then such H must be previously substituted by a suitable amine protecting group; and
b) isolating the supported proline derivative organocatalyst of formula I;
or alternatively,
a) reacting a support having a terminal alkyne or nitrile moiety and a proline derivative of formula IIb having a terminal azide moiety 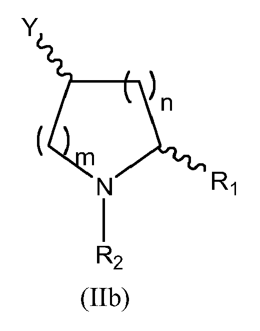 wherein the wavy line means any of the two possible configurations of the attached chiral atom; Y is selected from the group consisting of alkyl(C₁-C₁₀)-azide and azide; and wherein A, m, n, R₁ and R₂ are the same as defined above; provided that when R₁ is a reacting group containing an active moiety such as a -COOH, -CONH₂, or -OH, then the active moiety of R₁ must be previously protected with a suitable protecting group, and provided that when R₂ is H, then such H must be previously substituted by a suitable protecting group; and
b) isolating the supported proline derivative organocatalyst of formula I.

According to another aspect, the invention provides a process which comprises catalyzing an asymmetric reaction by addition of the supported proline derivative organocatalyst of the invention, which comprises reacting a prochiral or chiral compound in the presence of the catalyst according to the invention, to produce an optically active enantiomerically pure compound. This aspect of the invention may be formulated as the use of the compound (I) as organicatalyst for performing an asymmetric reaction

### DETAILED DESCRIPTION OF THE INVENTION:

As used herein, the following terms are used to describe the present invention. The definitions provided below, within context, may be used exclusively, or may be used to supplement definitions which are generally known to those of ordinary skill in the art.

The term "(C₁-C₆)alkyl" as used herein refers to a saturated branched or linear hydrocarbon chain with 1 to 6 hydrocarbon atoms. Preferably "(C₁-C₆)alkyl" is an unsubstituted group selected from methyl, ethyl, propyl, isopropyl, butyl, isobutyl, s-butyl and t-butyl.

The term "stereoisomers" refers to compounds which have identical chemical constitution, but differ with regard to the arrangement of their atoms or groups in space. In particular, the term "enantiomers" refers to two stereoisomers of a compound which are non superimposable mirror images of one another. The term "diastereomers", on the other hand, refers to the relationship between a pair of stereoisomers that comprise two or more asymmetric centers and are not mirror images of one another.

Furthermore, a "stereoselective process" is one which produces a particular stereoisomer of a reaction product in preference to other possible stereoisomers of that product. An "enantioselective process" is one which favors production of one of the two possible enantiomers of a reaction product.

In the context of this invention, the "enantiomerically pure" means a compound with enough enantiomeric excess for its preparation to industrial scale, which depends on each particular case as it would be decided by the person skilled in the art. For most purposes, a enantiomeric excess higher than 95% is enough. When the term enantiomerically pure is meant to indicate the ee of the catalyst, such ee is preferably higher than 99%.

The term "linker" is art-recognized and refers to a molecule or group of molecules connecting a support, including a solid support or polymeric support, and the active unit. The linker may be comprised of a single linking molecule or may comprise a linking molecule and a spacer molecule, intended to separate the linking molecule and the active unit by a specific distance.

The term "polymeric support" is art-recognized and refers to a soluble or insoluble polymer onto which the active unit is anchored directly or through a linker. Many suitable polymeric supports are known, and include soluble polymers such as polyethylene glycols or polyvinyl alcohols, as well as insoluble polymers such as polystyrene resins. A polymeric support is termed "soluble" if the polymer, or the polymer-supported compound, is soluble under the conditions employed. However, in general, a soluble polymer can be rendered insoluble under defined conditions. Accordingly, a polymeric support may be soluble under certain conditions and insoluble under other conditions.

The term "protecting group" refers to a chemical moiety or group which protects or prevents an active moiety or group from participating with or interfering with one or more chemical synthetic steps and its removal restores the moiety to its original active state. The term protecting group as used herein refers to those groups intended to protect against undesirable reactions during synthetic procedures. Such protecting groups are well known to those skilled in the art. Examples of protecting groups can be found in Green et al.,"Protective Groups in Organic Chemistry", (Wiley,2nd ed. 1991), J.F.W. McOmie et al. "ProtectiveGroups in Organic Chemistry", (Plenam Press, New York, 1973), and Harrison et al, "Compendium of Synthetic Organic Methods", Vols.1-8 (John Wiley and Sons, 1971-1996). Protecting groups can be removed with inter alia acid, base, fluoride ions, hydrogenation, metals such as zinc as well as by numerous other methods which are well known in the art. One of ordinary skill in the art can readily choose an appropriate protecting group to facilitate synthetic reactions according to method aspects of the present invention without engaging in undue experimentation. Representative amino protecting groups include, but are not limited to, formyl, acetyl, trifluoroacetyl, benzyl, benzyloxycarbonyl ("CBz"), tert-butoxycarbonyl ("BOC"), trimethylsilyl ("TMS"), 2-trimethylsilylethanesulfonyl ("SES"), trityl and substituted trityl groups, allyloxycarbonyl,9-fluorenylmethyloxycarbonyl("FMOC"), nitro-veratryloxycarbonyl("NVOC") and the like. Representative hydroxy protecting groups include, but are not limited to, those where the hydroxy group is either acylated or alkylated such as benzyl, and trityl ethers as well as alkyl ethers, tetrahydropyranyl ethers, trialkylsilyl ethers and allyl ethers. Preferably BOC, FMOC, CBz and orthonitrobenzyl carbamate groups may be used in the present invention. BOC group is a preferred group for the protection of nitrogen.

The term "solvent" is used to describe a medium (typically, but not necessarily inert) in which a reaction takes place using the organocatalyst according to the present invention. Solvents may include polar and non-polar solvents, including, for example, H₂O, pyridine, triethanolamine, tetrahydrofuran (THF), 1,4-dioxane, acetonitrile, dimethylacetamide (DMA), dimethylformamide (DMF), dimethylsulfoxide (DMSO), acetonitrile, methylene chloride, nitromethane, chloroform, methanol, ethanol, isopropanol, N-methylpyrrolidone (NMP), ethylacetate, benzene, toluene, etc. and mixtures thereof. Preferably, asymmetric reactions catalyzed by the supported proline derivative catalyst of the invention takes place using DMF, DMSO, THF, water or mixtures thereof as solvent.

It is clear to a person skilled in the art that the catalyst of the present invention have at least two optical centers and to thus form "stereoisomers". All optical isomers are part of the present invention and are thus encompassed by the scope of the claims.

In the molecular structures herein, the use of bold and dashed lines to denote particular configuration of groups follows the IUPAC convention. A bond indicated by a broken line indicates that the group in question is below the general plane of the molecule as drawn (the "alpha" configuration), and a bond indicated by a bold line indicates that the group at the position in question is above the general plane of the molecule as drawn (the "beta" configuration).

According to an embodiment of the first aspect of the invention, it relates to a supported proline derivative organocatalysts according to formula I, as defined above, wherein R₁ is selected from the group consisting of-COR₃, -CONHR₄, cycloalkyl(C₃-C₈), haloalkyl(C₁-C₆), -O-alkyl(C₁-C₆),-alkyl(C₁-C₆)-OH, -alkyl(C₁-C₆)-O-alkyl(C₁-C₆), aryl(C₆-C₁₈), phenylalkyl(C₁-C₆), biphenylalkyl(C₁-C₆), alkyl(C₁-C₆)biphenyl, substituted alkyl(C₁-C₆)biphenyl, phenylalkenyl(C₂-C₆), morpholyl, morpholylalkyl(C₁-C₆), pyrrolyl, substituted pyrrolyl, isoxazolyl, substituted isoxazolyl, imidazolyl, substituted imidazolyl, indolyl, substituted indolyl, pyrazolyl, substituted pyrazolyl, piperazinyl, piperidinyl, triazolyl, triazolylalkyl(C₁-C₆), tetrazolyl, tetrazolylalkyl(C₁-C₆), diarilhydroxypirrolidonyl, prolinolyl, alkyl(C₁-C₆)prolinol ether, pyridyl and substituted pyridyl; R₃ is selected from the group consisting of H, OH, linear or branched alkyl(C₁-C₆), -O-alkyl(C₁-C₆), aryl(C₆-C₁₈), pyrrolyl, substituted pyrrolyl, imidazolyl, substituted imidazolyl, pyrazolyl, substituted pyrazolyl, triazolyl, triazolylalkyl(C₁-C₆), tetrazolyl, tetrazolylalkyl(C₁-C₆), -alkyl(C₁-C₆)-O-alkyl(C₁-C₆), morpholyl, morpholylalkyl(C₁-C₆), piperazinyl, piperidinyl, diarilhydroxypirrolidonyl, prolinolyl, alkyl(C₁-C₆)prolinol ether, pyrrolidinyl, pyridyl and substituted pyridyl; and R₄ is selected from the group consisting of hydrogen, pyridyl and substituted pyridyl; the substituents being a radical independently selected from the group consisting of: halogen, (C₁-C₄)-alkyl, (C₁-C₄)-alkoxy, and hydroxy.

According to another embodiment of the first aspect of the invention, it relates to a supported proline derivative catalyst according to formula I, as defined above, wherein R₁ is selected from the group consisting of -COOH, -COO-alkyl(C₁-C₆), -CONH₂, alkyl(C₁-C₆)-OH, -alkyl(C₁-C₆)-O-alkyl(C₁-C₆), and

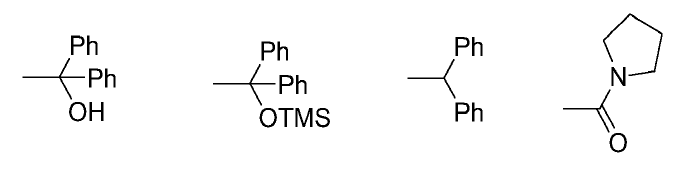

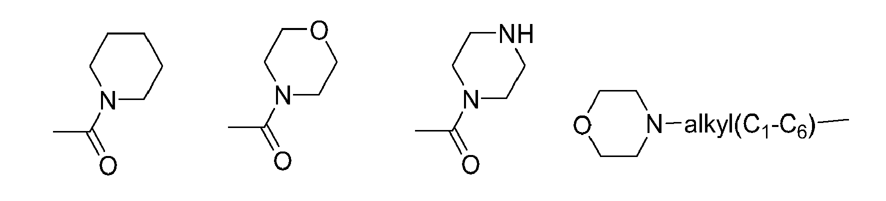

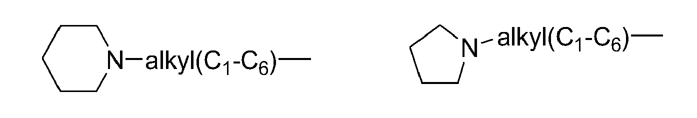

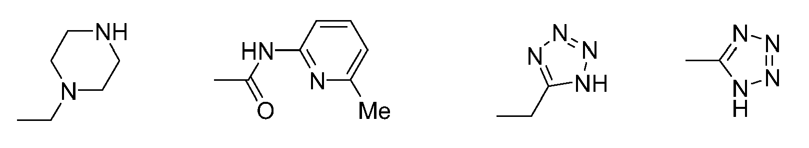

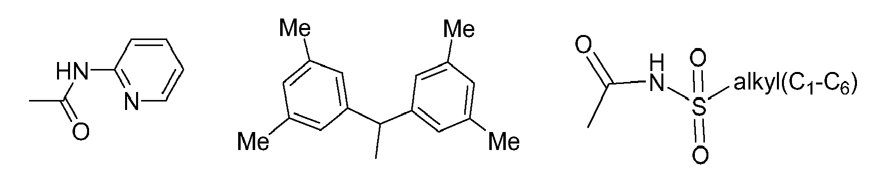

In a more preferred embodiment, R₁ is selected from the group consisting of

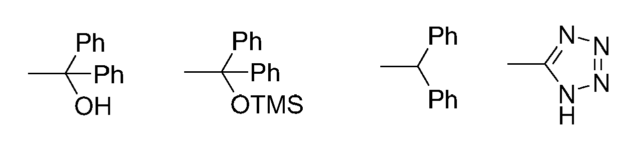

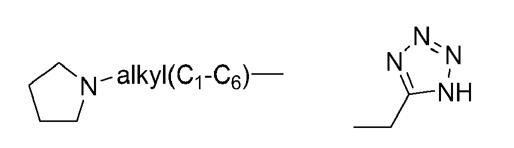

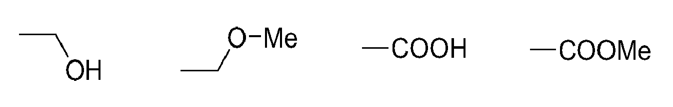

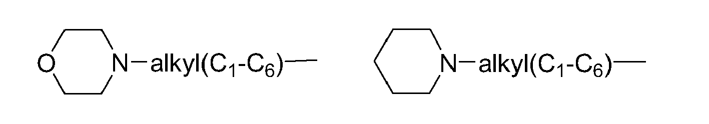

Particularly preferred are those wherein R₁ is -COOH. Furthermore preferably, R₁ is -COOH and R₂ is H. Furthermore preferred compounds of the general formula I are those wherein m = 1 and n = 1.

In a preferred embodiment, A and B are biradicals independently selected from the group consisting of -alkyl(C₁-C₃)-, -O-alkyl(C₁-C₃)-, -alkyl(C₁-C₃)-O-, and -alkyl(C₁-C₃)-O-alkyl(C₁-C₃)-. More preferably, A and B are independently selected from the group consisting of -O-CH₂-, -CH₂-O-, -CH₂-, -CH₂-O-CH₂-, -CH₂-CH₂-CH₂-, -CH₂-CH₂-, -O-CH₂-CH₂-CH₂-, -O-CH₂-CH₂-, -CH₂-CH₂-CH₂-O-, -CH₂-CH₂-O-, and -CH₂-CH₂-O-CH₂-CH₂-. Particularly preferred, A and B are independently selected from the group consisting of -O-CH₂, -CH₂-O-, -CH₂-, and -CH₂-O-CH₂-.

According to a preferred embodiment, it relates to a catalyst according to formula Ia 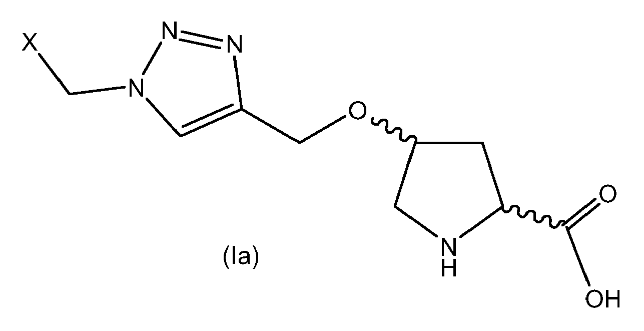 wherein the wavy line means any of the two possible configurations of the attached chiral atom and X represents the polymeric support.

In the catalyst of formula (I) the active unit for asymmetric reactions is the proline derivative moiety of formula (II) 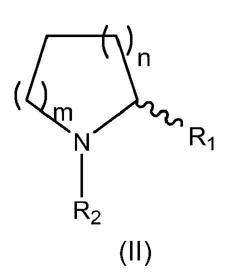 wherein the wavy line means any of the two possible configurations of the attached chiral atom; m, n, R₁ and R₂ are the same as defined above.

Particularly preferred compounds of formula (I) are those of *trans-*(2S,4R) configuration, i.e. those of formula (III) 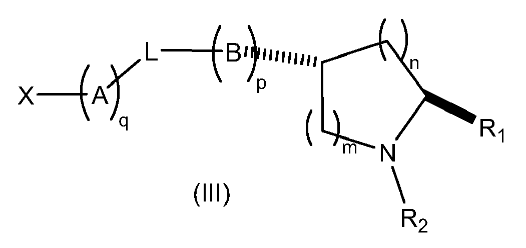 wherein n, m, p, q, A, B, L, X, R₁ and R₂ are the same as defined above, and the bold bond and the dashed bond denotes the particular configuration of the molecule *(trans-(2S,4R)).*

Accordingly, particularly preferred embodiments of the invention relates to the preferred embodiments defined above wherein furthermore are of *trans-(2S,4R)* configuration.

Therefore, in a more specific embodiment of the present invention, it relates to compounds of formula (III) wherein m, n, p, and q are equal to 1, A is CH₂, B is -OCH₂-, and L is 1,4-triazole, according to formula (IIIa)

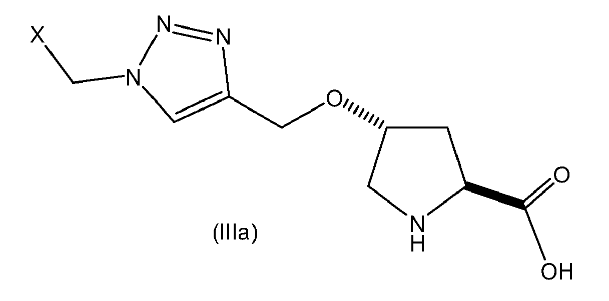

Particularly preferred is a catalyst according to the invention in which the linker may be obtained by a 1,3-dipolar cycloaddition reaction. More preferably, the 1,3-dipolar cycloaddition occurs though an azide-substituted support with a o-propargyl hydroxyproline derivative.

The support is a polymer, in particular a polystyrene resin (PS), a polyacrylate, a polyacrylamide, a polyvinylpyrrolidinone, a polysiloxane, a polybutadiene, a polyisoprene, a polyalkane, a polyoxazoline, a polyether, or mixtures thereof. Particularly preferred is a polystyrene resin, and more preferred a Merrifield resin, the Argopore® resin and a PS-PEG resin. Even more preferred, the Merrifield 1% DVB or 2% DVB is used due to their high hydrophobicity, since resin swelling would likely be key for catalysis.

The beneficial contribution of the catalyst to the overall outcome of the asymmetric reaction is shown by an enhanced of the *anti:syn* ratio compared to the monomer of formula (IV). In experiments using the monomer of formula IV as catalyst, the *anti*:syn ratio was reduced to 63:37, and ee for the anti diastereomer was only 83%.

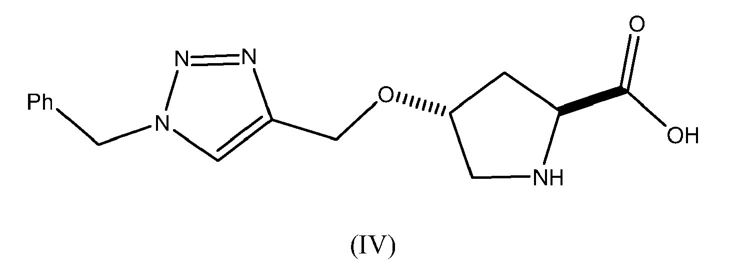

According to the second aspect, the invention provides a method for the preparation of the supported proline derivative organocatalysts of the invention. Particularly preferred is the method which comprises:
a) reacting a support having a terminal azide moiety and a proline derivative of formula IIa having a terminal alkyne or nitrile moiety 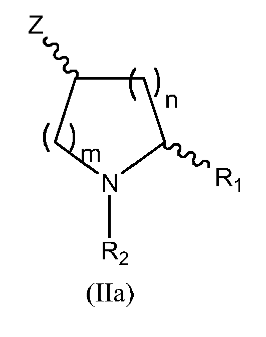 wherein the wavy line means any of the two possible configurations of the attached chiral atom; Z is selected from the group consisting of alkyne(C₃-C₁₀) or alkyl(C₁-C₁₀)-CN; and wherein m, n, R₁ and R₂ are the same as defined above, provided that when R₁ is a reacting group containing an active moiety such as a -COOH, -CONH₂, or -OH, then the active moiety of R₁ is previously protected with a suitable protecting group, and provided that when R₂ is H, then such H must be previously substituted by a suitable amine protecting group;
b) isolating the supported proline derivative organocatalysts of formula I.

According to another preferred embodiment, the method comprises:
a) reacting a support having a terminal alkyne or nitrile moiety and an proline derivative of formula IIb having a terminal azide moiety 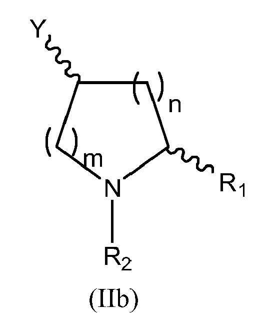 wherein the wavy line means any of the two possible configurations of the attached chiral atom; Y is selected from the group consisting of alkyl(C₁-C₁₀)-azide and azide; and wherein m, n, R₁ and R₂ are the same as defined above , provided that when R₁ is a reacting group containing an active moiety such as a -COOH, -CONH₂, or -OH, then the active moiety of R₁ must be previously protected with a suitable protecting group, and provided that when R₂ is H, then such H must be previously substituted by a suitable amine protecting group;
b) isolating the supported proline derivative organocatalysts of formula I.

According to the present invention, the "terminal azide support", "terminal alkyne support" and "terminal nitrile support" refers to a support suitably functionalized with an azide, alkyne or nitrile moiety according to the following formulas: 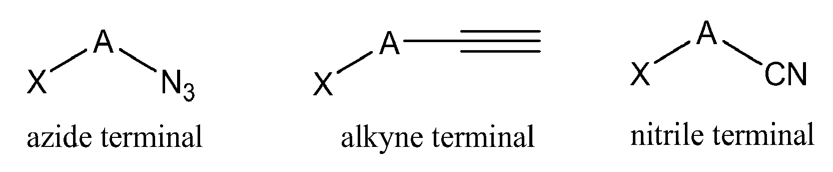 wherein X and A are the same as defined above.

The formation of triazoles via the cycloaddition of azide and acetylene was first reported by Dimroth in the early 1900's, but the generality, scope, and mechanism of these cycloadditions was not fully realized until the 1960's. The Huisgen 1,3-dipolar cycloaddition between a terminal alkyne and an azide has rapidly become the most popular click reaction to date (Huisgen, R. Proc. Chem. Soc. 1961, pp. 357). The term "click chemistry" was introduced by K.B. Sharpless (Angew. Chemie, Inter. Ed. 2001, vol. 40, pp. 2004-2021) and relates to a group of high-yield chemical reactions leading to small molecules in a very fast, clean, and predictable manner.

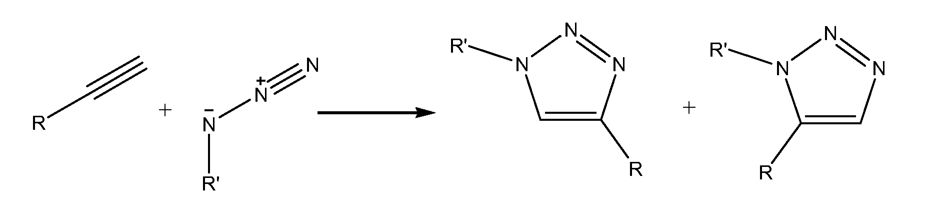

The reaction generates a mixture of 1,4- and 1,5-disubstituted triazoles, as various attempts to control the regioselectivity have been reported without much success until the discovery of the copper(I)-catalyzed reaction in 2002, which exclusively yields the 1,4-disubstituted 1,2,3-triazole.

In addition to triazoles, tetrazoles may also be prepared via the highly regioselective [2+3] cycloaddition, in this case between an organic azide (R-N₃) and an organic nitrile (R'-CN) (Angew Chem Ind Ed 2002, vol. 41, pp. 2110-2113.)

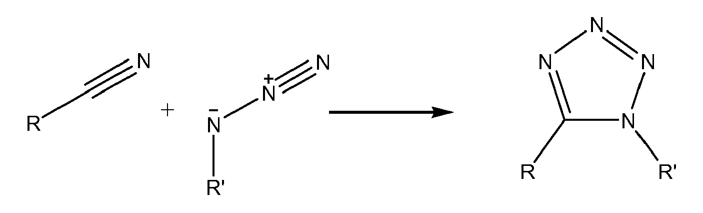

The immobilization of TEMPO (2,2,6,6-tetramethylpiperidine-1-oxyl) by copper-catalyzed azide-alkyne [3+2] cycloaddition (1,3-dipolar cycloaddition reaction) onto polystyrene resin (PS-click-TEMPO) is described in A. Gheorghe et al.; Adv. Synth. Catal. 2006, vol. 348, pp.1016-1020. The so obtained PS-click-TEMPO product is used as a catalyst in the oxidation of alcohols to aldehydes.

More preferably, the proline derivative of formula IIa having a terminal alkyne moiety is that of formula (IIa-1): 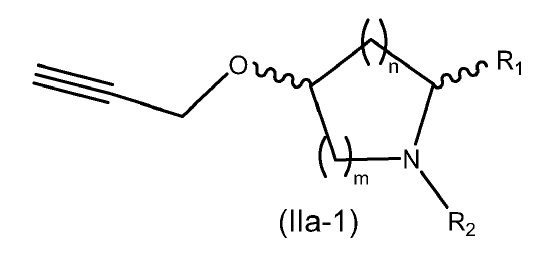 wherein the wavy line means any of the two possible configurations of the attached chiral atom; n, m, R₁ and R₂ are as defined above.

In a more preferred embodiment of the invention, the proline derivative of formula IIa having a terminal alkyne moiety is that of formula (IIa-2):

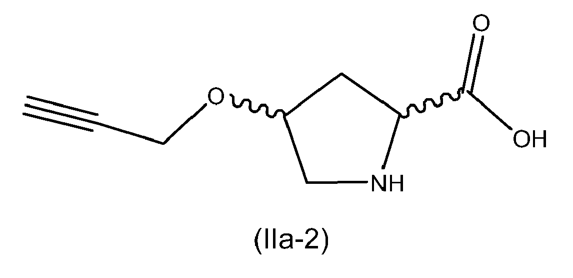

When the method for the preparation of the supported proline derivative organocatalyst of the invention occurs through the reaction of the proline derivative of formula IIb, preferably such proline derivative is that of formula (IIb-1) 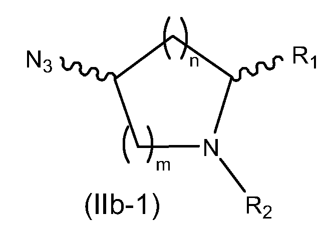 wherein the wavy line means any of the two possible configurations of the attached chiral atom;n, m, R₁ and R₂ are as defined above.

More preferably, is that of formula (IIb-2)

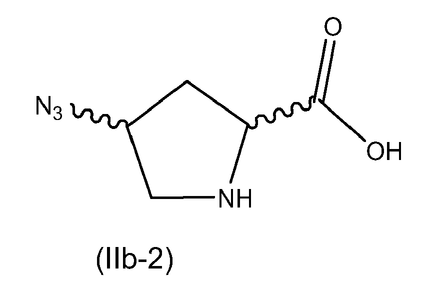

According to a preferred embodiment, in the preparation process the proline derivative of formula IIa or IIb is of *trans-(2S,4R)* configuration.

In a preferred embodiment. the reaction of the azido-terminal moiety with the alkyl-terminal moiety or nitrile-terminal moiety takes place under 1,3-dipolar cycloaddition conditions. Preferably, that reaction is catalyzed by an addition of a catalytic amount of Cu(I) or by an addition of Cu(II) in the presence of a reducing agent for reducing said Cu(II) to Cu(I), *in situ,* in catalytic amount.

Accordingly, the 1,3-dipolar cycloaddition reaction step to anchor the active unit to the support is a key step in the formation of the catalyst due to its advantages with respect to common S_{N}2 anchoring. This strategy offers the advantages of being compatible with the introduction of structural diversity on the monomer and of increasing spatial separation between the polymer backbone and the active site, while no negative effects on reactivity or stereocontrol were expected from the rather inert 1,2,3-triazole moiety. Furthermore, 1,3-dipolar cycloaddition reactions results in the formation of anchoring of two individual moieties with very high yields, no by-products generated, and high stereospecifity.

According to an embodiment of the invention, the preparation process of the catalyst results in an enantiomerically pure catalyst. Therefore, the invention refers to a catalyst according to formula (I) in any enantiomerically pure form. Preferably, the catalyst obtained has an enantiomeric excess of the *trans*-(2S,4R) enantiomer greater than 90%. More preferably, the catalyst obtained has an ee of the *trans-*(2S,4R) enantiomer greater than 99%.

According to another aspect, the invention provides a process which comprises catalyzing an asymmetric reaction by addition of supported proline derivative organocatalysts of the invention, in catalytic amount.

Preferably, the asymmetric reaction is a Mannich reaction, aldol condensation reaction, tandem Mukaiyama Aldol- cyclization reaction, tandem Mannich-Michael reaction, Michael addition reaction, hydroxyamination reaction, asymmetric Robinson annulation, asymmetric cross coupling of aldehydes, asymmetric synthesize sugars, α-amination reaction, α-aminooxylation of aldehydes and ketones, S_{N}2 alkylation, [4+2] addition, the Morita-Baylis-Hillman reaction, selenenylation and or-sulfenylation reactions of aldehydes and ketones and the Michael addition reaction of aldehydes to nitroolefins. More preferably, the asymmetric reaction is an aldol condensation reaction, a hydroxyamination reaction, and a Mannich type reaction.

Proline and proline derivatives has been found to be a efficient chiral organocatalyst in several reactions:
i) Asymmetric Robinson Annulation (Hajos, Z. G.; Parrish, D. R.; J. Org. Chem. 1974, 39, 1615): 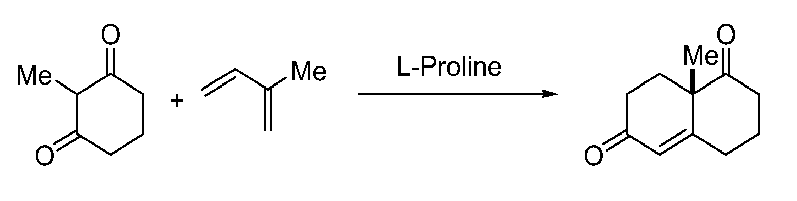
ii) Asymmetric aldol reaction (Benaglia, M. et al. Adv. Synth Catal. 2002, 344, 533-542): 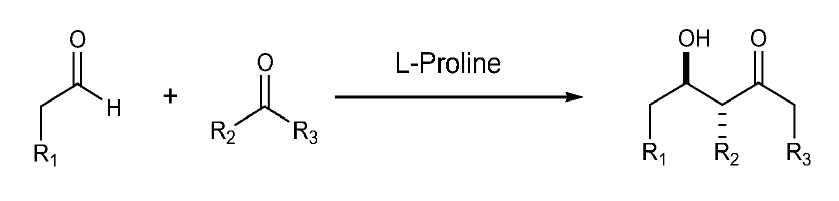
iii) Asymmetric Cross coupling of aldehydes (Hayashi, Y.; Aratake, S.; et al. Angew. Chem. Int. Ed. 2006 ASAP): 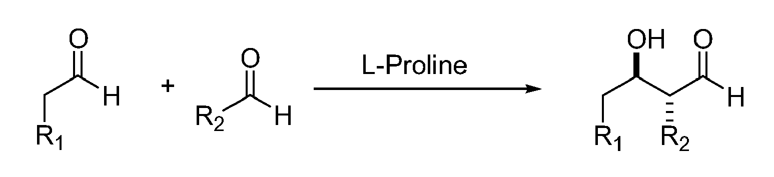
iv) Asymmetric Synthesize sugars (Córdova, A.; Ibrahem, I.; Casa, J.; Engqvist, M.; Kaynak, B.; Angew. Chem. Int. Ed. 2005, 44, 1343): 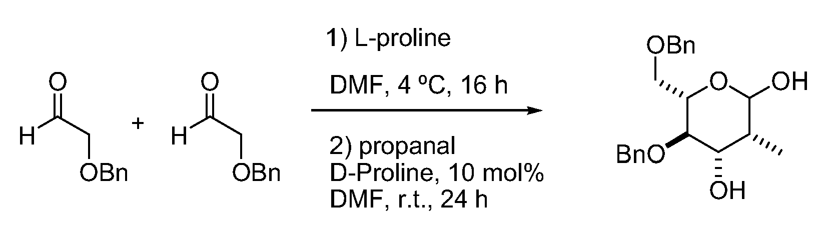
v) Asymmetric Michael Reaction (Hanessian, S.; Govindan, S.; warrier, J.S. Chirality 2005, 17, 540): 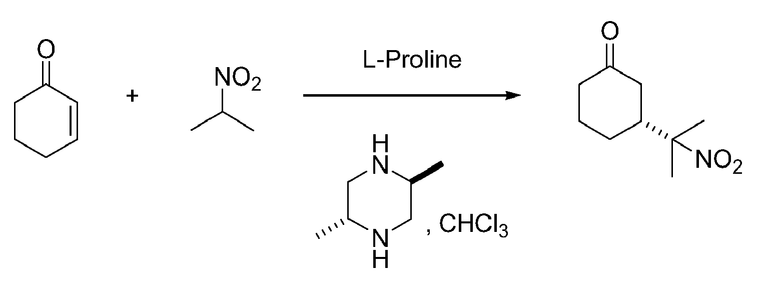
vi) Asymmetric Mannich Reaction (Hayashi, Y.; Sakai, K. Angew. Chem Int. Ed. 2003, 42, 3677): 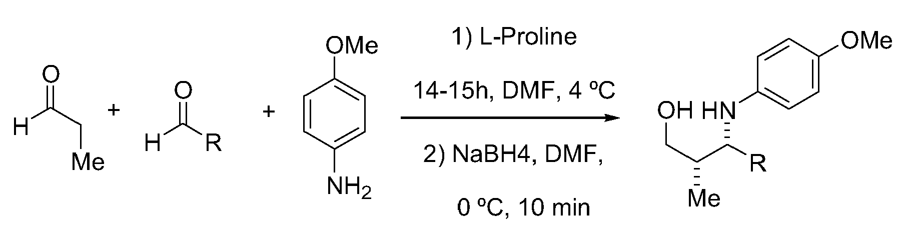
vii) Asymmetric tandem Mannich-Michael reaction (Córdova, A. Synlett 2003, 1651-1654): 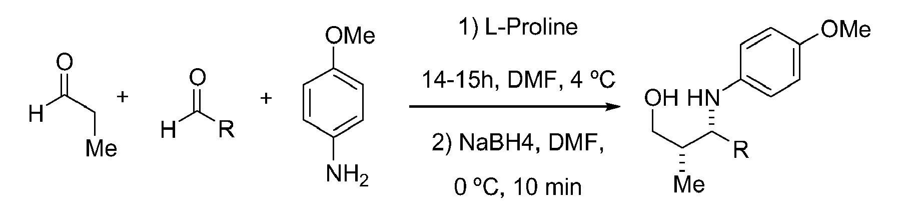
viii) α-Amination reaction (Barbas, C. F. III; Steiner, D. D.; Suri, J. T. Org. Lett. 2005, 7, 3885): 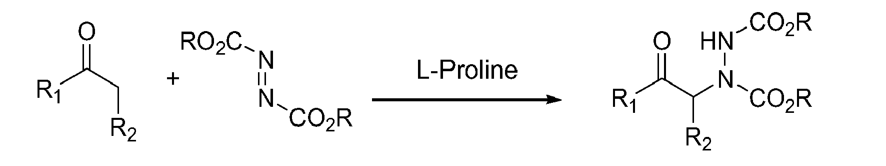 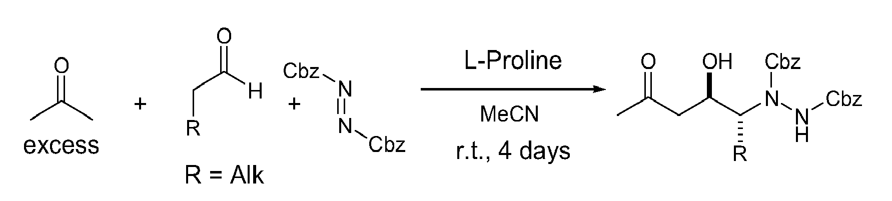
ix) α-Aminooxylation of Aldehydes and ketones(MacMillan, D. W. C.; Sinz, C. J.; Brouch, M. P.; Brown, S. P. J. Am. Chem. Soc. 2003, 125, 10808): 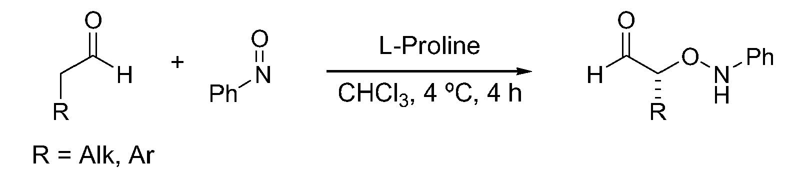 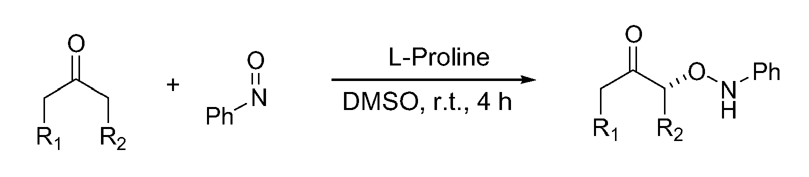
x) S_{N}2 Alkylation (Dalko, P. I.; Moisan, L. Angew. Chem Int. Ed. 2004, 43, 5138-5175): 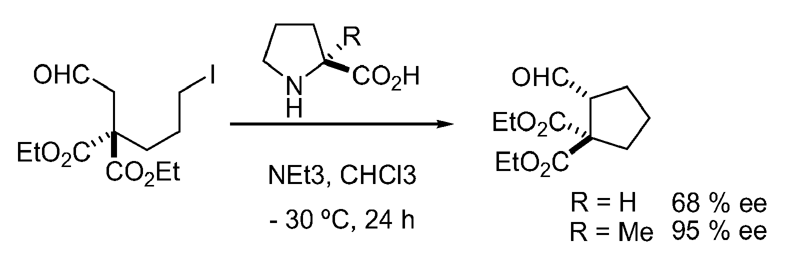
xi) [4+2] Addition (Dalko, P. I.; Moisan, L. Angew. Chem Int. Ed. 2004, 43, 5138-5175): 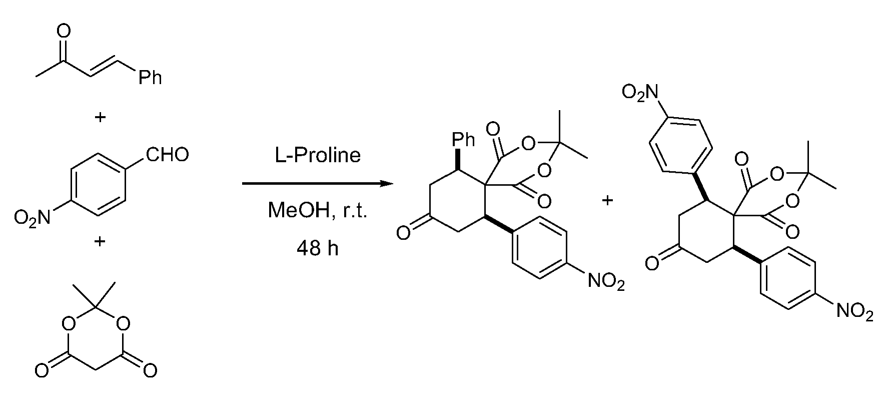
xii) The Morita-Baylis-Hillman Reaction (Dalko, P. I.; Moisan, L. Angew. Chem Int. Ed. 2004, 43, 5138-5175): 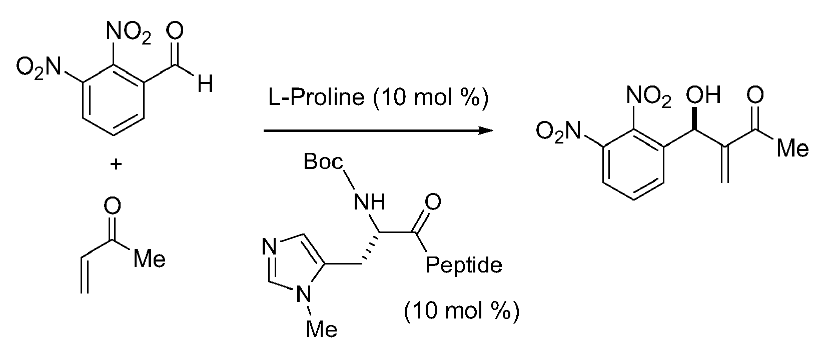 organocatalysts of the invention, as those cited above, can be carried out in accordance with conventional procedures known in the art.

Therefore, the catalytist according to the invention may be used to catalyze those reactions, in many instances with high stereo- and/or enantiomeric selectivity, with good yield, and wherein the catalyst may be efficiently recycled and reused without loss of activity.

In a preferred embodiment, the process for performing an asymmetric reaction which comprises reacting a prochiral or chiral compound in the presence of the catalyst according to the invention to produce an optically active product, is an aldol condensation, a hydroxyamination or a Mannich type reaction.

Accordingly, the invention more specifically relates to a method for performing asymmetric proline or proline derivatives catalyzed reactions, that give high diastereoselectivity and high enantiomeric enrichment (ee), According to a preferred embodiment, such high diastereoselectivity and high enantiomeric enrichment is preferably for the major anti diastereomer. Furthermore, surprisingly, the asymmetric reactions catalyzed by the supported proline derivative organocatalyst of the invention, takes place in an aqueous medium without the use of organic solvents with high diastereoselectivity and high enantiomeric enrichment (ee) for the major *anti* diastereomer. An additional advantage is that yield losses associated with the production of an undesired enantiomer can be substantially reduced.

According to a preferred embodiment of this aspect of the invention, an catalytic amount of an additive is added to the reaction media. Preferably, the additive is a surfactant. More preferably is DiMePEG (Dimethylpolyethylenglycol).

Throughout the description and claims the word "comprise" and variations of the word, such as "comprising", are not intended to exclude other technical features, additives, components, or steps.

Additional objects, advantages and features of the invention will become apparent to those skilled in the art upon examination of the description or may be learned by practice of the invention. The following examples and drawings are provided by way of illustration, and is not intended to be limiting of the present invention.

### DETAILED DESCRIPTION OF PARTICULAR EMBODIMENTS

### Example 1. Procedure for the synthesis of (azidomethyl)polystyrene

Sodium azide (0.78 g, 51 mmol) was added to a mixture of 3 g of (Chloromethyl)polystyrene (f = 0.6 ~ 0.8 mmol/g) in 30 mL of DMSO. The mixture was headed for 18 h at 60°C. After cooling the suspension was filtrated and the resin was washed with water (500 mL), THF (250 mL), THF-MeOH 1:1 (250 mL), MeOH (250 mL) and THF (250 mL). The solid was dried in vacuo for 24 hours at 40 °C.
IR (ATR) = 2093 cm⁻¹.
Elemental analysis (%) = N 3.17, C 89.14, H 8.38.
f = 0.75 mmol/g.

### Example 2. Procedure for the synthesis of polystyrene-supported proline derivative catalyst, (IIIa)

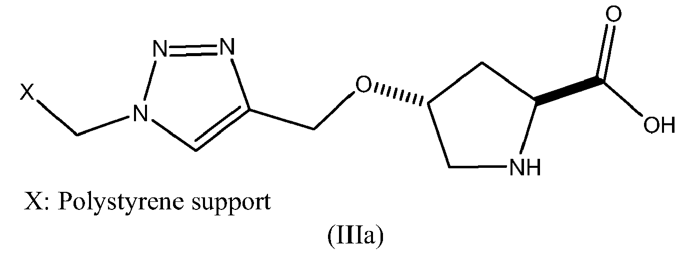

### a) Cycloaddition:

(2S,4R)-di-tert-butyl-4-(prop-2-ynylloxy)pyrrolidine-1,2-dicarboxylate, (0.16 g, 0.5 mmol), N,N-Diisopropylethylamine (0.86 mL, 4.9 mmol) and copper (I) iodide (0.006 g, 0.003 mmol) were added to a suspension of (azidomethyl)polystyrene (0.5 g, f = 0.75 mmol/g) and DMF:THF 1:1 (10 mL) at 35 °C for 24 h. The reaction mixture was monitored and once the IR-signal of the azido group had completely disappeared, the resin was collected by filtration and washed with water (250 mL), DMF (250 mL), THF (250 mL), THF-MeOH 1:1 (250 mL), MeOH (250 mL) and THF (250 mL). The solid was dried in vacuo for 24 hours at 40 °C.
IR (ATR) = 2974 (C-H), 1739 (C=O), 1701 (C=O), 1402 (tBu), 1391 (tBu) cm⁻¹. Elemental analysis (%) = N 3.42, C 84.23, H 8.54.
f = 0.61 mmol/g.

### b) Deprotection

1 g (f = 0.61 mmol/g) cycloaddition resin was swelled with 5 mL of CH2Cl2, after 10 minutes 10 mL of trifluoroacetic acid was added. The reaction mixture was filtrated when the IR-signal of tBu and carbonyl group had completely disappeared. Polysyterene-supported proline, 1, was washed with THF (with 2 % of Et₃N, 250 mL), water (250 mL), THF (250 mL), THF-MeOH 1:1 (250 mL), MeOH (250 mL) and THF (250 mL). The solid was dried in vacuo for 24 hours at 40 °C
IR (ATR) = 2920 (C-H), 1600 (C=O) cm⁻¹.
Elemental analysis (%) = N 3.39, C 79.76, H 7.85.
f = 0.61 mmol/g.

### Example 3. Synthesis of di-tert-butyl-4-(prop-2-ynyloxy)pyrrolidine-1,2-dicarboxylate.

A solution of tert-butyl (2S,4R)-N-Boc-4-hydroxyprolinate (0.61 g, 2.1 mmol) in DMF (5 mL) was added a via canula to a suspension of sodium hydride (0.082 g, 3.2 mmol) in DMF (5 mL) at -20 °C under N2. The mixture was stirred for 20 min, and propargyl bromide (0.35 mL, 3.2 mmol) was syringed into the mixture. After being stirred for 1 h at -20 °C, the mixture was allowed to reach room temperature, and stirred for 16 h. MeOH was added (2 mL), and the reaction mixture was extracted with CH₂Cl₂ (3x10 mL), and washed with brine (5 mL). The combined organic extracts were dried and concentrated in vacuo. Purification by silica gel column chromatography (EtOAc/hexane 2:8) gave (0.43g, 63 %) as an oil.
¹H-NMR (400 MHz, CDCl₃): δ = 1.45 (s, 18H), 2.07 (m, 1 H), 2.33 (m, 1 H), 2.44 (t, 1 H, J1 = 2.33 Hz), 3.50 (m, 1 H), 3.62 (m, 1 H), 4.13 (m, 2H), 4.24 (m, 2H) ppm.
¹³C-NMR (100 MHz, CDCl3): δ = 28.0 (CH3), 28.3 (CH₃), 36.6 (CH₂), 51.0 (CH₂), 56.4(CH), 58.5(CH2), 74.7 (≡CH), 75.6 (≡C), 79.2 (CH), 80.1 (C), 81.2 (C), 153.9 (C=O), 172.0 (C=O) ppm.
IR (ATR): v = 3247 (=CH), 2976 (C-H), 1738 (C=O), 1698 (C=O), 1399 (tBu), 1366 (tBu) cm⁻¹.
HRMS (CI+): m/z = 326.1956, calculated for C₂₇H₂₈NO₅ 326.1967 (M+H)
[α]D29 °C = -45.7 (c = 3.52, CH₂Cl₂)

### Example 4. Synthesis of (2S,4R)-di-tert-butyl-4-((1-benzyl-1H-1,2,3-triazol-4-yl)methoxy)pyrrolidine-1,2-dicarboxylate (IV)

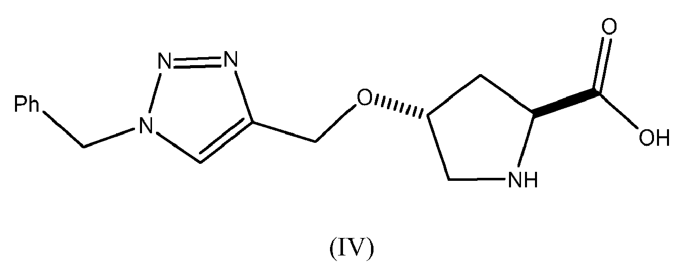

### a) Cycloadditon

Benzyl bromide (0.13 mL, 1.07 mmol) was added to a mixture of (2S,4R)-di-tert-butyl-4-(prop-2-ynylloxy)pyrrolidine-1,2-dicarboxylate (0.35 g, 1 mmol), sodium azide (0.148 g, 2.13 mmol), copper (II) sulfate pentahydrate (0.005 g, 0.02 mmol), and L-ascorbic acid, sodium salt (0.043 g, 0.21 mmol) in tert-butanol and water 1:1 (3 mL). The mixture was headed by microwaves (150 W, 100 °C, 2 min ramp and 20 min hold time). The mixture was extracted with ethyl acetate (3x25 mL), the combined organic phases were dried over anhydrous Na₂SO₄ and concentrated in vacuo after filtration. Purification by silica gel column chromatography (EtOAc/hexane 2:8) gave (*2S,4R*)-di-tert-butyl 4-((1-benzyl-1H-1,2,3-triazol-4-yl)methoxy)pyrrolidine-1,2-dicarboxylate (0.43 mg, 88 %) as a clear oil.
¹H-NMR (400 MHz, CDCl₃, 55°C): δ = 1.45 (s, 18H), 2.02 (m, 1 H), 2.30 (m, 1 H), 4.20 (m, 2H), 4.59 (s, 2H), 5.49 (s, 2H), 7.27 (m, 2H), 7.35 (m, 2H), 7.42 (s, 1 H) ppm.
¹³C-NMR (100 MHz, CDCl₃, 55°C): δ = 27.9 (CH₃), 28.3 (CH₃), 36.5 (CH₂), 51.5 (CH₂), 54.1 (CH), 58.6 (CH₂), 62.9 (CH₂), 76.2 (CH), 79.9 (C), 81.2 (C), 122.3 (CH), 128.0 (CH), 128.7 (CH), 129.0 (CH), 134.5 (C), 145.2 (C), 154.2 (C=O), 171.9 (C=O) ppm.
IR (ATR): v = 2976 (C-H), 1736 (C=O), 1694 (C=O), 1394 (tBu), 1365 (tBu) cm⁻¹.
HRMS (Cl +): m/z = found 481.2448, calculated for C₂₄H₃₄N₄O₅Na, 481.2427 (M+Na)
[α]D = -30.4 (c = 0.41, CHCl₃)

### b) Deprotection

(*2S,4R*)-di-tert-butyl 4-((1-benzyl-1H-1,2,3-triazol-4-yl)methoxy)pyrrolidine-1,2-dicarboxylate (0.25 g, 0.54 mmol) was dissolved with 3 mL of a solution of HCl in MeOH (1.25 M). After 1 h the mixture was concentrated in vacuo and a white solid precipitated. The solid was added to a 1:1 solution of TFA:CH₂Cl₂ (2.5 mL). After 1 h the mixture was concentrated in vacuo and dried to give (*2S,4R*)-4-((1-benzyl-1H-1,2,3-triazol-4-yl)methoxy)pyrrolidine-2-carboxylic acid, hydrochloride, in quantitative yield.
¹H-NMR (400 MHz, CDCl₃ 55°C): δ = 1.45 (s, 18H), 2.02 (m, 1 H), 2.30 (m, 1 H), 4.20 (m, 2H), 4.59 (s, 2H), 5.49 (s, 2H), 7.27 (m, 2H), 7.35 (m, 2H), 7.42 (s, 1 H) ppm.
¹³C-NMR (100 MHz, CDCl₃ 55°C): 27.9 (CH₃), 28.3 (CH₃), 36.5 (CH₂), 51.5 (CH₂), 54.1 (CH), 58.6 (CH₂), 62.9 (CH₂), 76.2 (CH), 79.9 (C), 81.2 (C), 122.3 (CH), 128.0 (CH), 128.7 (CH), 129.0 (CH), 134.5 (C), 145.2 (C), 154.2 (C=O), 171.9 (C=O) ppm.
IR (ATR): v = 2945 (C-H), 1736 (C=O), 1655, 1132 cm⁻¹.
HRMS (ESI +): m/z = found 303.1450, calculated for C₁₅H₁₉N₄O₃, 303.1457 (M-Cl).
[α]D 29 °C = -10.7 (c = 0.72, CH₂Cl₂)

### Example 5. Typical procedure for the synthesis of (2S,1'R)-2-(Hydroxyphenylmethyl)cyclohexan-1-one.

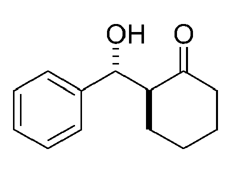

Catalyst (IIIa) (66 mg, 0.04 mmol) was added to a suspension of benzaldehyde (40.6 µL, 0.4 mmol), cyclohexanone (207 µL, 2.0 mmol) and DiMePEG (80 mg, MW 2000 g/mol) in water (0.14 mL) at room temperature. The reaction mixture was stirred for 60 h, after this time, 0.5 mL of ethyl acetate was added and the mixture was concentrated in vacuo after filtration and washing with ethyl acetate (4x2 mL). The mixture was purified by silica gel flash chromatography (EtOAc/hexane 2:8) to give 2-(hydroxyphenylmethyl)-cyclohexanone (60 mg, 75 %) as a clear oil.
Absolute stereochemistry was determined by the comparison with the literature data.
[α]D = +19.3 (c= 0.95, CHCl3), 96% ee.
Enantiomeric excess was determined by HPLC with a Chiralcel OD-H column (99:1 Hexane:2-propanol), 1 mL/min; λ = 213 nm; major enantiomer tr = 18.1 min, minor enantiomer tr = 28.5 min.
*Anti:syn* = 96:4 (by ¹H-NMR of the crude mixture)
All the spectroscopic data matched with literature.

A representative set of aldehydes and ketones were examined under the optimized reaction conditions (10 mol% of catalyst IIIa, 10 mol% DiMePEG, water, room temperature (r.t.) to check the scope of the catalyst. Results are shown in table 1. Aromatic aldehydes reacted with cyclohexanone with diastereoselectivities higher than those recorded for monomeric proline derivatives in water (Mase, N.; et al. J. Am. Chem. Soc. 2006, 128, 734-735; Hayashi, Y.; et al. Angew. Chem., Int. Ed. 2006, 45, 958-961).

Following the next reaction scheme, different aldol reactions in water and catalyzed by 10 mol% of catalyst IIIa and DiMePEG as surfactant were obtained: 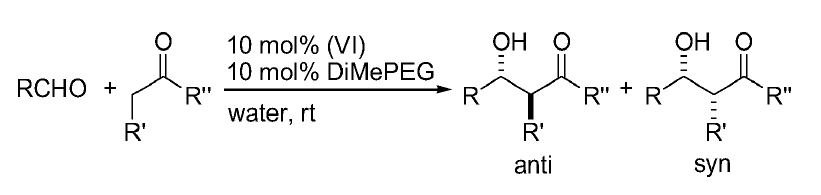
a) Isolated yield.
b) Determined by ¹H-NMR of the crude product.
c) Determined by HPLC using a chiral stationary phase.
d) 25 eq. of acetone was used.
e) Estimated by ₁H-NMR.

Product entry 2: Enantiomeric excess was determined by HPLC with a Chiralcel AS column (98:2 Hexane:2-propanol), 1 mL/min; λ = 254 nm; major enantiomer retention time (tᵣ) = 27.5 min, minor enantiomer tᵣ = 33.0 min. All the spectroscopic data matched with literature.

Product entry 3: Enantiomeric excess was determined by HPLC with a Chiralcel AD-H column (80:20 Hexane:2-propanol), 1 mL/min; λ = 254 nm; major enantiomer tᵣ = 23.7 min, minor enantiomer tᵣ = 30.2 min. All the spectroscopic data matched with literature.

Product entry 4: Enantiomeric excess was determined by HPLC with a Chiralcel OD-H column (95:5 Hexane:2-propanol), 0.25 mL/min; λ = 254 nm; major enantiomer tᵣ = 70.0 min, minor enantiomer tᵣ = 99.2 min. All the spectroscopic data matched with literature.

Product entry 5: Enantiomeric excess was determined by HPLC with a Chiralcel AS column (88:12 Hexane:2-propanol), 0.5 mL/min; λ = 254 nm; major enantiomer tᵣ = 19.5 min, minor enantiomer tᵣ = 21.1 min. All the spectroscopic data matched with literature.

Product entry 6: Enantiomeric excess was determined by HPLC with a Chiralcel OD-H column (95:5 Hexane:2-propanol), 1 mL/min; λ = 220 nm; major enantiomer tᵣ = 21.4 min, minor enantiomer tᵣ = 23.5 min. All the spectroscopic data matched with literature.

Product entry 7: Enantiomeric excess was determined by HPLC with a Chiralcel OD-H column (95:5 Hexane:2-propanol), 1 mL/min; λ = 213 nm; major enantiomer tᵣ = 11.4 min, minor enantiomer tᵣ = 14.3 min. All the spectroscopic data matched with literature.

Product entry 8: Enantiomeric excess was determined by HPLC with a Chiralcel OJ column (90:10 Hexane:2-propanol), 1 mL/min; λ = 254 nm; major enantiomer tᵣ = 32.8 min, minor enantiomer tᵣ = 37.3 min. All the spectroscopic data matched with literature.

Product entry 9: Enantiomeric excess was determined by HPLC with a Chiralcel AS column (90:10 Hexane:2-propanol), 1 mL/min; λ = 214 nm; major enantiomer tᵣ = 19.8 min, minor enantiomer tᵣ = 28.9 min. All the spectroscopic data matched with literature.

Product entry 10: Enantiomeric excess was determined by HPLC with a Chiralcel OD-H column (90:10 Hexane:2-propanol), 1 mL/min; λ = 213 nm; major enantiomer tᵣ = 11.0 min, minor enantiomer tᵣ = 13.3 min. All the spectroscopic data matched with literature.

**Table 1. Aldol reaction in water catalyzed by 10 mol% of 1 and DiMePEG**

| Entry | Product | Yield^{a} [%] | t [h] | anti: syn^{b} | ee^{c} [%] |
|---|---|---|---|---|---|
| 1 | 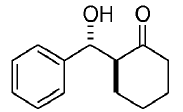 | 75 | 60 | 96:4 | 96 |
| 2 | 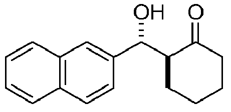 | 70 | 84 | 95:5 | 95 |
| 3 | 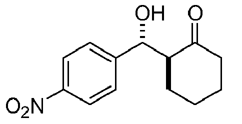 | 85 | 18 | 98:2 | 97 |
| 4 | 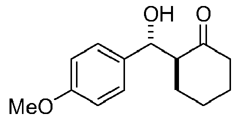 | 18 | 108 | 90:10 | 93 |
| 5 | 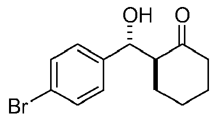 | 90 | 84 | 96:4 | 95 |
| 6 | 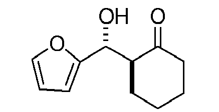 | 92 | 65 | 82:18 | 94 |
| 7 | 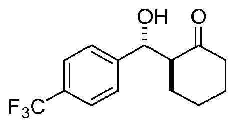 | 97 | 60 | 97:3 | 96 |
| 8^{d} | 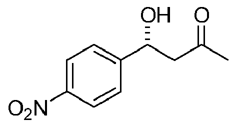 | 24 | 60 | - | 59 |
| 9 | 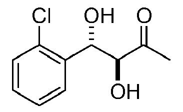 | 42^{e} | 144 | 58:42 | 45 |
| 10 | 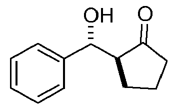 | 97 | 60 | 83:17 | 87 |

Moreover, ee for the major isomers practically did not deteriorate with respect to the monomeric catalysts. In difficult examples involving other ketones (entries 8-10), a similar behavior was observed. In addition to these characteristics, the robustness of resin IIIa is noteworthy. Thus, after quenching the reactions the resin is simply washed with AcOEt and simply dried for reuse. No decrease in its performance is observed after three uses of a same sample and, in fact, results in Table 1 have been obtained with recycled samples of catalyst of formula IIIa.

### Example 6. Recycling of polystyrene-supported proline derivative catalyst.

The polystyrene-supported proline derivative catalyst IIIa was recycled and reused in asymmetric aldol reaction. The following table 2 contains the results of yield %, *anti:syn* ratio and ee of *anti* and *syn.*

**Table 2. Recycling of the catalyst.**

| Entry | cycle | Yield[%] | anti:syn | anti ee[%] | syn ee[%] |
|---|---|---|---|---|---|
| 1 | 1 | 75 | 96:4 | 96 | 75 |
| 2 | 2 | 87 | 95:5 | 96 | 58 |
| 3 | 3 | 81 | 95:5 | 96 | 72 |
| 4 | 4 | 65 | 95:5 | 94 | 21 |

### Example 7. Effect of DiMePEG on the aldol reaction catalyzed by (IIIa) in water.

In order to improve the aldol yield, reaction time was increased and a catalytic amount of water-soluble DiMePEG (MW ~2000) was added with the hope of facilitating difusion to the resin.

Results of the catalyzed aldol reaction in presence of DiMePEG are summarized in table 3

**Table 3. DiMePEG effect on the aldol reaction catalyzed by product IIIa (10 mol%) in water.**

| Entry | Additive [10 mol%] | Yield^{a} [%] | t [h] | *anti:syn*^{b} [%] | ee *anti*^{c} |
|---|---|---|---|---|---|
| 1 | none | 67 | 84 | 95:5 | 95 |
| 2 | DiMePEG | 46 | 18 | 95:5 | 96 |
| 3 | DiMePEG | 75 | 60 | 96:4 | 96 |
| 4 | DiMePEG^{d} | 70 | 60 | 92:8 | 91 |

| | | | | | |
|---|---|---|---|---|---|
| a) Isolated yield. b) Determined by 1 H-NMR of the crude product. c) Determined by HPLC using a chiral stationary phase. d) 5 mol% of catalyst IIIa was used. | | | | | |

Clearly, improved yields can be obtained at shorter reaction times (compare entries 1 and 3, and entry 2 with entry 1 in table 4). Reducing the amount of catalytic resin IIIa to 5 mol% still allowed to isolate the aldol in good yield, but with somewhat diminished stereocontrol (entry 4).

### Example 8. Solvent effect on the aldol reaction catalyzed by catalyst IIIa.

Following the method of example 5, the aldol reaccion of cyclohexanone with benzaldehyde was carried out with different solvents and solvent-water mixtures. The results are summarized in table 4.

**Table 4. Solvent effect on the aldol reaction catalyzed by resin IIIa.**

| Entry | Solvent | Yield^{a} [%] | *anti:syn*^{b} | ee *anti^{c}* [%] | ee *syn*^{c} [%] |
|---|---|---|---|---|---|
| 1 | water | 26 | 95:5 | 96 | 61 |
| 2 | DMF^{d} | 90 | 50:50 | 44 | 87 |
| 3 | DMF:water 94:6 | 30 | 84:16 | 92 | 90 |
| 4 | DMF:water 71:29 | 48 | 93:7 | 96 | 84 |
| 5 | DMF:water 50:50 | 28 | 95:5 | 96 | 85 |
| 6 | DMSO^{d} | 95 | 50:50 | 84 | 89 |
| 7 | DMSO:water 94:6 | 90 | 82:18 | 91 | 90 |
| 8 | DMSO:water 71:29 | 73 | 91:9 | 95 | 95 |
| 9 | DMSO:water 50:50 | 55 | 93:7 | 96 | 87 |
| 10 | neat | <5 | n.d. | n.d. | n.d. |

| | | | | | |
|---|---|---|---|---|---|
| a) Isolated yield. b) Determined by 1 H-NMR of the crude product. c) Determined by HPLC using a chiral stationary phase. d) Synthesis grade. | | | | | |

The reaction worked nicely in water, yielding the aldol product in high diastereoselectivity and high ee for the major anti diastereomer (entry 1). On the other hand, the diastereoselectivity was lost and ee for anti diastereomers deteriorated when good resin-swelling solvents like DMF and DMSO (entries 2 and 10) were used in the reaction. However, overall yield increased noticeably in these reactions. Increasing the water content in these solvents improved the diastereo- and enantioselectivities, but at the expense of lower reactivity. Without solvent, the reaction proceeded sluggishly (entry 10). Thus, water appears as the optimal media for the reaction, and this strongly suggests that the reaction takes place at the interface between the polymer and the aqueous phase.

## Claims

1. An enantiomerically pure compound of formula (I), or a salt thereof, or a N-oxide thereof, or a solvate thereof, 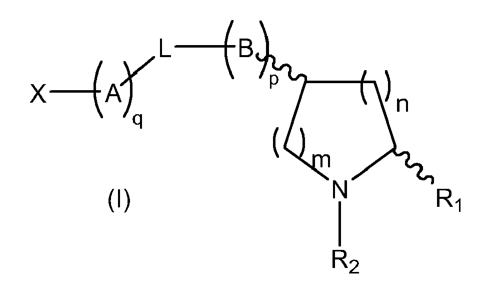 wherein:
the wavy line means any of the two possible configurations of the attached chiral atom;
X represents the polymeric support;
m and n are independently an integer from 0 to 4;
p and q are independently an integer from 0 to 1;
L is a biradical selected from the group consisting of 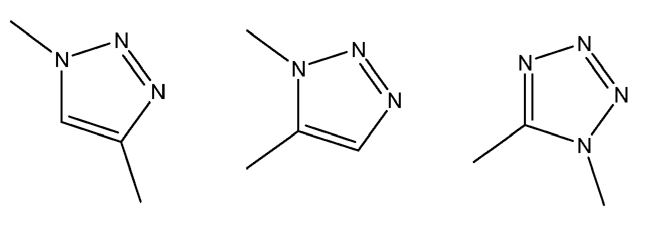
wherein L may be linked to A and B in its two possible positions;
A and B are biradicals independently selected from the group consisting of -alkyl(C₁-C₁₀)-, -O-alkyl(C₁-C₁₀)-, -alkyl(C₁-C₁₀)-O-, and -alkyl(C₁-C₁₀)-O-alkyl(C₁-C₁₀)-;
R₁ is selected from the group consisting of -COR₃, -CONHR₄, cycloalkyl(C₃-C₈), haloalkyl(C₁-C₆), -O-alkyl(C₁-C₆), -alkyl(C₁-C₆)-OH, -alkyl(C₁-C₆)-O-alkyl(C₁-C₆), aryl(C₆-C₁₈), phenylalkyl(C₁-C₆), biphenylalkyl(C₁-C₆), alkyl(C₁-C₆)phenyl, alkyl(C₁-C₆)biphenyl, substituted alkyl(C₁-C₆)biphenyl, phenylalkenyl(C₂-C₆), napthyl, substituted napthyl, napthylalkyl(C₁-C₆), napthylaikenyl(C₂-C₆), morpholyl, morpholylalkyl(C₁-C₆), furyl, substituted furyl, benzofuryl, substituted benzofuryl, pyrrolyl, substituted pyrrolyl, isoxazolyl, substituted isoxazolyl, benzoisoxazolyl, substituted benzoisoxazolyl, imidazolyl, substituted imidazolyl, benzimidazolyl, substituted benzimidazolyl, indolyl, substituted indolyl, pyrazolyl, substituted pyrazolyl, thienyl, substituted thienyl, benzothienyl, substituted benzothienyl, thiazolyl, substituted thiazolyl, benzothiazolyl, substituted benzothiazolyl, quinolinyl, substituted quinolinyl, isoquinolinyl, substituted isoquinolinyl, piperazinyl, piperidinyl, triazolyl, triazolylalkyl(C₁-C₆), tetrazolyl, tetrazolylalkyl(C₁-C₆), diarilhydroxypirrolidonyl, prolinolyl, alkyl(C₁-C₆)prolinol ether, pyridyl and substituted pyridyl;
R₂ is selected from the group consisting of hydrogen and linear or branched alkyl(C₁-C₆);
R₃ is selected from the group consisting of H, OH, linear or branched alkyl(C₁-C₆), alkenyl(C₂-C₆), alkynyl(C₂-C₆), cycloalkyl(C₃-C₈), haloalkyl(C₁-C₆), -O-alkyl(C₁-C₆), aryl(C₆-C₁₈), phenylalkyl(C₂-C₆), furyl, substituted furyl, pyrrolyl, substituted pyrrolyl, isoxazolyl, substituted isoxazolyl, imidazolyl, substituted imidazolyl, indolyl, substituted indolyl, pyrazolyl, substituted pyrazolyl, thienyl, substituted thienyl, thiazolyl, substituted thiazolyl, quinolinyl, substituted quinolinyl, isoquinolinyl, substituted isoquinolinyl, triazolyl, triazolylalkyl(C₁-C₆), tetrazolyl, tetrazolylalkyl(C₁-C₆), -alkyl(C₁-C₆)-O-alkyl(C₁-C₆), morpholyl, morpholylalkyl(C₁-C₆), piperazinyl, piperidinyl, diarilhydroxypirrolidonyl, prolinolyl, alkyl(C₁-C₆)prolinol ether, pyrrolidinyl, pyridyl and substituted pyridyl; and
R₄ is selected from the group consisting of hydrogen, linear or branched alkyl(C₁-C₆), cycloalkyl(C₃-C₆), pyridyl, substituted pyridyl, and alkyl(C₁-C₆)sulfonyl;
the substituents being a radical independently selected from the group consisting of: halogen, (C₁-C₄)-alkyl, (C₁-C₄)-alkoxy, and hydroxy.

2. The catalyst according to claim 1, wherein
R₁ is selected from the group consisting of -COR₃, -CONHR₄, cycloalkyl(C₃-C₈), haloalkyl(C₁-C₆), -O-alkyl(C₁-C₆), -alkyl(C₁-C₆)-OH, -alkyl(C₁-C₆)-O-alkyl(C₁-C₆), aryl(C₆-C₁₈), phenylalkyl(C₁-C₆), biphenylalkyl(C₁-C₆), phenylalkenyl(C₂-C₆), morpholyl, morpholylalkyl(C₁-C₆), pyrrolyl, substituted pyrrolyl, isoxazolyl, substituted isoxazolyl, imidazolyl, substituted imidazolyl, indolyl, substituted indolyl, pyrazolyl, substituted pyrazolyl, piperazinyl, piperidinyl, triazolyl, triazolylalkyl(C₁-C₆), tetrazolyl, tetrazolylalkyl(C₁-C₆), diarilhydroxypirrolidonyl, prolinolyl, alkyl(C₁-C₆)prolinol ether, pyridyl and substituted pyridyl;
R₃ is selected from the group consisting of H, OH, linear or branched alkyl(C₁-C₆), -O-alkyl(C₁-C₆), aryl(C₆-C₁₈), pyrrolyl, substituted pyrrolyl, imidazolyl, substituted imidazolyl, pyrazolyl, substituted pyrazolyl, triazolyl, triazolylalkyl(C₁-C₆), tetrazolyl, tetrazolylalkyl(C₁-C₆), -alkyl(C₁-C₆)-O-alkyl(C₁-C₆), morpholyl, morpholylalkyl(C₁-C₆), piperazinyl, piperidinyl, diarilhydroxypirrolidonyl, prolinolyl, alkyl(C₁-C₆)prolinol ether, pyridyl and substituted pyridyl; and
R₄ is selected from the group consisting of hydrogen, pyridyl and substituted pyridyl;
the substituents being a radical independently selected from the group consisting of: halogen, (C₁-C₄)-alkyl, (C₁-C₄)-alkoxy, and hydroxy.

3. The catalyst according to any of claims 1 to 2, wherein R₁ is selected from the group consisting of -COOH, -COO-alkyl(C₁-C₆), -CONH₂, alkyl(C₁-C₆)-OH, -alkyl(C₁-C₆)-O-alkyl(C₁-C₆),

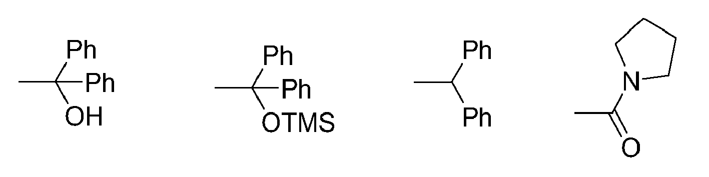

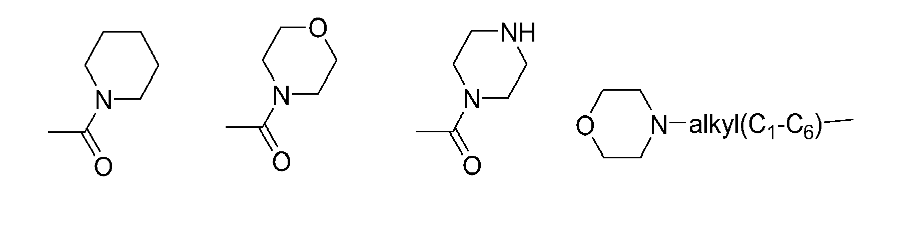

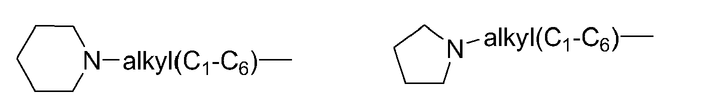

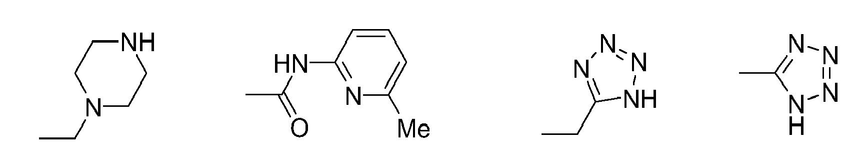

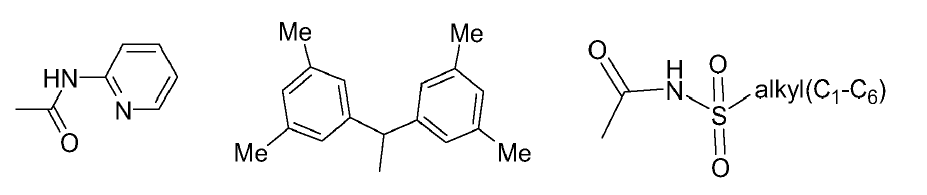

4. The catalyst according to any of claims 1 to 3, wherein R₁ is selected from the group consisting of

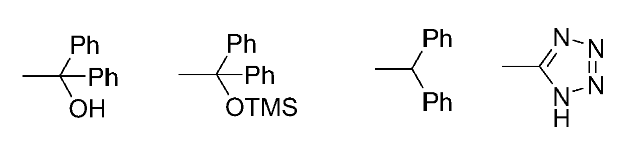

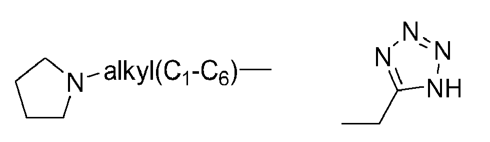

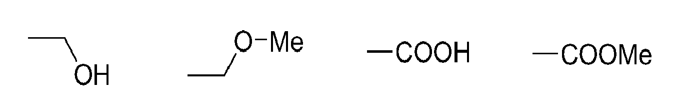

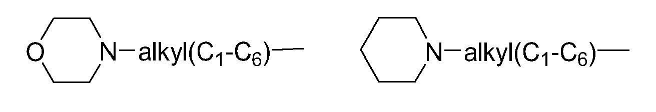

5. The catalyst according to any of claims 1 to 4, wherein R₁ is -COOH.

6. The catalyst according to any of claims 1 to 5, wherein R₂ is H.

7. The catalyst according to any of claims 1 to 6, wherein m = 1 and n = 1.

8. The catalyst according to any of claims 1 to 7, wherein A and B are biradicals independently selected from the group consisting of -alkyl(C₁-C₃)-, -O-alkyl(C₁-C₃)-, -alkyl(C₁-C₃)-O-, and -alkyl(C₁-C₃)-O-alkyl(C₁-C₃)-.

9. The catalyst according to any of claims 1 to 8, wherein B and D are independently selected from the group consisting of -O-CH₂-, -CH₂-O-, -CH₂-, and -CH₂-O-CH₂-.

10. The catalyst according to any of claims 1 to 9, of formula Ia

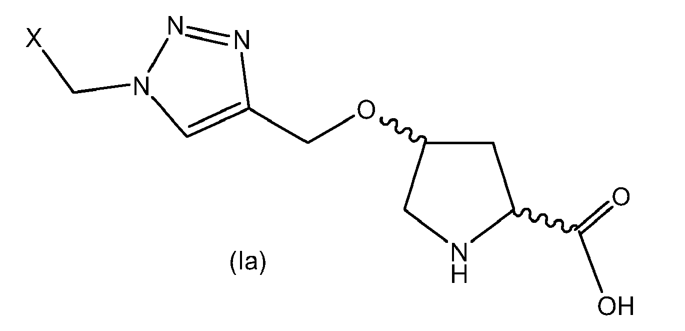

11. The catalyst according to any of claims 1 to 10, which is of *trans-(2S,4R)* configuration according to formula III

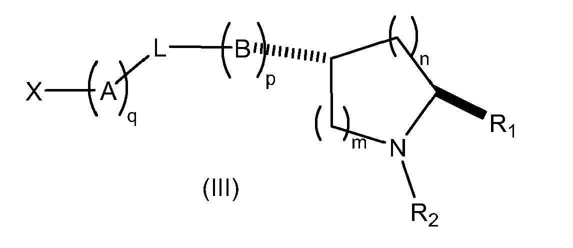

12. The catalyst according to claim 11, of formula IIIa

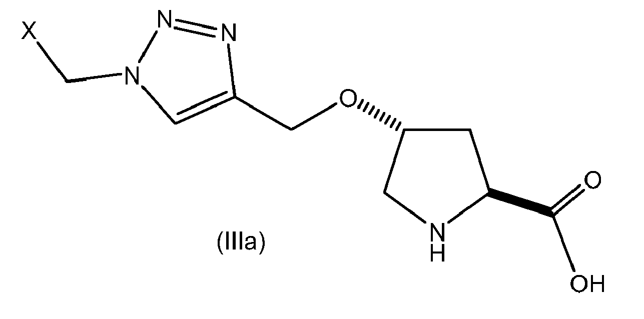

13. A preparation process of a compound of formula I, as defined in any of the claims 1-12, which comprises:
a) reacting a support having a terminal azide moiety and a proline derivative of formula IIa having a terminal alkyne or nitrile moiety 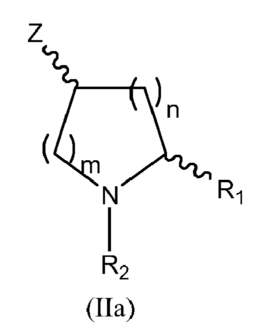 wherein the wavy line means any of the two possible configurations of the attached chiral atom; Z is selected from the group consisting of alkyne(C₃-C₁₀) or alkyl(C₁-C₁₀)-CN; and wherein A, m, n, R₁ and R₂ are the same as defined in any of preceding claims 1 to 11, provided that when R₁ is a reacting group containing an active moiety such as a -COOH, -CONH₂, or - OH, then the active moiety of R₁ must be previously protected with a suitable protecting group, and provided that when R₂ is H, then such H must be previously substituted by a suitable amine protecting group;
b) isolating the supported proline derivative organocatalysts of formula I.

14. A preparation process of a compound of formula I, as defined in any of the claims 1-12, which comprises:
a) reacting a support having a terminal alkyne or nitrile moiety and an proline derivative of formula IIb having a terminal azide moiety 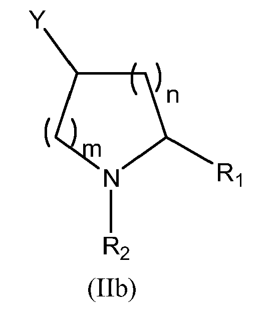 wherein Y is selected from the group consisting of alkyl(C₁-C₁₀)-azide and azide; and wherein A, m, n, R₁ and R₂ are the same as defined in any of preceding claims 1 to 11, provided that when R₁ is a reacting group containing an active moiety such as a -COOH, -CONH₂, or -OH, then the active moiety of R₁ must be previously protected with a suitable protecting group, and provided that when R₂ is H, then such H must be previously substituted by a suitable amine protecting group;
b) isolating the supported proline derivative organocatalysts of formula I.

15. The preparation process according to any of claims 13 or 14, wherein the reaction is catalyzed by an addition of a catalytic amount of Cu(I) or by an addition of Cu(II) in the presence of a reducing agent for reducing said Cu(ll) to Cu(I), *in situ* in catalytic amount.

16. The preparation process according to claim 15, wherein the proline derivative of formula IIa or IIb is of *trans-*(*2S,4R*) configuration.

17. A process for performing an asymmetric reaction which comprises reacting a prochiral or chiral compound in the presence of the catalyst according to any of claims 1 to 12, to produce an optically active product.

18. The process according to claim 17 wherein the asymmetric reaction is selected from the group consisting of a Mannich, aldol condensation, tandem Mukaiyama Aldol- cyclization, tandem Mannich-Michael, Michael addition, hydroxyamination, asymmetric Robinson annulation, asymmetric cross coupling of aldehydes, asymmetric synthesis of sugars, α-amination, α-aminooxylation of aldehydes and ketones, S_{N}2 alkylation, [4+2] addition, the Morita-Baylis-Hillman, selenenylation or sulfenylation of aldehydes and ketones and the Michael addition of aldehydes to nitroolefins reaction.

19. The process according to any of claims 17 to 18, wherein the asymmetric reaction is an aldol condensation, a hydroxyamination or a Mannich type reaction.
